(19) Europäisches Patentamt
European Patent Office
Office européen des brevets

(11) EP 2 019 090 B1

(12) EUROPEAN PATENT SPECIFICATION

(45) Date of publication and mention of the grant of the patent:
**14.11.2012 Bulletin 2012/46**

(21) Application number: **07721123.3**

(22) Date of filing: **14.05.2007**

(51) Int Cl.:
*C07C 235/34* (2006.01) *C07D 213/79* (2006.01)
*C07D 213/80* (2006.01) *C07D 317/60* (2006.01)
*C07D 405/12* (2006.01) *C07C 69/732* (2006.01)

(86) International application number:
**PCT/CN2007/001550**

(87) International publication number:
**WO 2007/131446 (22.11.2007 Gazette 2007/47)**

(54) **SUBSTITUTED BETA-PHENYL-ALPHA-HYDROXY PROPANOIC ACID, SYNTHESIS METHOD AND USE THEREOF**

SUBSTITUIERTE BETA-PHENYL-ALPHA-HYDROXYPROPANSÄURE, SYNTHESEVERFAHREN UND ANWENDUNG DAVON

ACIDE BÊTA-PHÉNYL-ALPHA-HYDROXY PROPANOÏQUE SUBSTITUÉ, PROCÉDÉ DE SYNTHÈSE ET SON UTILISATION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **15.05.2006 CN 200610042787**

(43) Date of publication of application:
**28.01.2009 Bulletin 2009/05**

(60) Divisional application:
**12002538.2 / 2 514 739**

(73) Proprietor: **Northwest University**
**Xi'an, Shaanxi 710069 (CN)**

(72) Inventors:
- **ZHENG, Xiaohui**
**Xi'an, Shaanxi 710069 (CN)**
- **ZHANG, Qunzheng**
**Xi'an, Shaanxi 710069 (CN)**
- **WANG, Shixiang**
**Xi'an, Shaanxi 710069 (CN)**
- **ZHAO, Xinfeng**
**Xi'an, Shaanxi 710069 (CN)**

(74) Representative: **Maiwald, Walter**
**Maiwald Patentanwalts GmbH**
**Elisenhof**
**Elisenstrasse 3**
**80335 München (DE)**

(56) References cited:
**WO-A-03/002575 FR-A- 2 842 523**

- **DATABASE BEILSTEIN BEILSTEIN INSTITUTE FOR ORGANIC CHEMISTRY, FRANKFURT-MAIN, DE; XP002559338 Database accession no. BRN2102386 & ASPELUND: ACTA ACADEMIAE ABOENSIS, SERIES B, MATHEMATICA ET PHYSICA, vol. 10, no. 14, 1937, pages 13-36,**
- **DATABASE BEILSTEIN BEILSTEIN INSTITUTE FOR ORGANIC CHEMISTRY, FRANKFURT-MAIN, DE; XP002559339 Database accession no. BRN1949612 & VAN DER STELT ET AL: RECUEIL DES TRAVAUX CHIMIQUES DES PAYS-BAS, vol. 92, 1973, pages 493-512,**
- **DATABASE BEILSTEIN BEILSTEIN INSTITUTE FOR ORGANIC CHEMISTRY, FRANKFURT-MAIN, DE; XP002559340 Database accession no. BRN2677217 & MORREN: CHEM.ABSTRACTS, vol. 56, 1962, page 2329,**
- **DATABASE BEILSTEIN BEILSTEIN INSTITUTE FOR ORGANIC CHEMISTRY, FRANKFURT-MAIN, DE; XP002559341 Database accession no. BRN2387441 & GEFFKEN ET AL: ARCHIV DER PHARMAZIE, vol. 319, no. 7, 1986, pages 577-582,**
- **DATABASE BEILSTEIN BEILSTEIN INSTITUTE FOR ORGANIC CHEMISTRY, FRANKFURT-MAIN, DE; XP002559342 Database accession no. BRN3291354 & ASPELUND: ACTA ACADEMIAE ABOENSIS, SERIES B, MATHEMATICA ET PHYSICA, vol. 11, no. 2, 1937, page 15,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- DATABASE BEILSTEIN BEILSTEIN INSTITUTE FOR ORGANIC CHEMISTRY, FRANKFURT-MAIN, DE; XP002559343 Database accession no. BRN6394184 & GOWAL ET AL: HELVETICA CHIMICA ACTA, vol. 68, 1985, pages 2132-2139,
- DATABASE BEILSTEIN BEILSTEIN INSTITUTE FOR ORGANIC CHEMISTRY, FRANKFURT-MAIN, DE; XP002559344 Database accession no. BRN4541943 & OJIMA ET AL: TETRAHEDRON LETTERS, vol. 21, 1980, pages 3907-3910,
- DATABASE BEILSTEIN BEILSTEIN INSTITUTE FOR ORGANIC CHEMISTRY, FRANKFURT-MAIN, DE; XP002559345 Database accession no. BRN4557169 & OJIMA ET AL: TETRAHEDRON LETTERS, vol. 21, 1980, pages 3907-3910,
- DATABASE BEILSTEIN BEILSTEIN INSTITUTE FOR ORGANIC CHEMISTRY, FRANKFURT-MAIN, DE; XP002559346 Database accession no. BRN5065337 & OJIMA ET AL: CHEMISTRY LETTERS, 1980, pages 853-856,
- DATABASE BEILSTEIN BEILSTEIN INSTITUTE FOR ORGANIC CHEMISTRY, FRANKFURT-MAIN, DE; XP002559347 Database accession no. BRN5130401 & OJIMA ET AL: CHEMISTRY LETTERS, 1980, pages 853-856,
- DATABASE BEILSTEIN BEILSTEIN INSTITUTE FOR ORGANIC CHEMISTRY, FRANKFURT-MAIN, DE; XP002559348 Database accession no. BRN6607392 & WUENSCH ET AL: TETRAHEDRON ASYMMETRY, vol. 4, no. 11, 1993, pages 2307-2310,
- DATABASE BEILSTEIN BEILSTEIN INSTITUTE FOR ORGANIC CHEMISTRY, FRANKFURT-MAIN, DE; XP002559349 Database accession no. BRN6630225 & HARADA ET AL: TETRAHEDRON LETTERS, vol. 34, no. 38, 1993, pages 6091-6094,
- DATABASE BEILSTEIN BEILSTEIN INSTITUTE FOR ORGANIC CHEMISTRY, FRANKFURT-MAIN, DE; XP002559350 Database accession no. BRN7242845 & MURAKAMI ET AL: TETRAHEDON LETTERS, vol. 36, no. 16, 1995, pages 2785-2788,
- DATABASE BEILSTEIN BEILSTEIN INSTITUTE FOR ORGANIC CHEMISTRY, FRANKFURT-MAIN, DE; XP002559351 Database accession no. BRN7294414 & NAGATSU ET AL: CHEMICAL &PHARMACEUTICAL BULLETIN, vol. 43, no. 5, 1995, pages 887-889,
- DATABASE BEILSTEIN BEILSTEIN INSTITUTE FOR ORGANIC CHEMISTRY, FRANKFURT-MAIN, DE; XP002559352 Database accession no. BRN7532496 & UCHIDA ET AL: JOURNAL OF ANTIBIOTICS, vol. 49, no. 9, 1996, pages 886-889,
- DATABASE BEILSTEIN BEILSTEIN INSTITUTE FOR ORGANIC CHEMISTRY, FRANKFURT-MAIN, DE; XP002559353 Database accession no. BRN8381662 & ISHIDA ET AL: TETRAHEDRON, vol. 55, no. 36, 1999, pages 10971-10988,
- H.JOHNSON ET AL: JOURNAL OF ORGANIC CHEMISTRY, vol. 28, 1963, pages 3255-3256, XP009126875
- ANDRUS M.B. ET AL.: 'Asymmetric Phase-Transfer Catalyzed Glycolate Alkylation, Investigation of the Scope, and Application to the Synthesis of (-)-Ragaglitazar' JOURNAL OF ORGANIC CHEMISTRY vol. 70, no. 23, 2005, pages 9470 - 9479, XP003024722
- DALKO P.I. ET AL.: 'Stereoselective hetero-Claisen rearrangement of camphor derived oxazoline-N-oxides' TETRAHEDRON LETTERS vol. 39, no. 15, 1998, pages 2107 - 2110, XP003024723
- GAMBONI R. ET AL.: 'Structure and diastereoselectivity of the alpha-hydroxylation of chiral ester enolates by molybdenum peroxo complex' TETRAHEDRON LETTERS vol. 27, no. 34, 1986, pages 3999 - 4002, XP003024724

## Description

### Technical Field

The present invention relates to a substituted β-phenyl-α-hydroxyl-propionic acid derivative, a process for synthesizing the same, and use thereof for the manufacture of a medicament for treatment and prevention of cardiovascular and cerebrovascular diseases.

### Background Art

Dan-shen Root (*Radix Salviae Militiorrhizae*) is a traditional Chinese medicine for treatment of cardiovascular and cerebrovascular diseases with definite therapeutic effects. At present, it is generally presumed that salvianic acid (chemical name: β-(3,4-dihydroxyphenyl)-α-hydroxyl-propionic acid) is the main active ingredient in the water-soluble components of Dan-shen Root. It is demonstrated in Pharmacological tests that β-phenyl-α-hydroxylpropionic acid is the pharmacologically active portion in propanoid acid, but its potency is not desirable. Thus, the substituted β-phenyl-α-hydroxylpropionic acid was modified, and the resulting modified derivatives might possess the same or greater potency than the parent compound, and might also exhibit an improved therapeutic effect in the treatment and prevention of cardiovascular and cerebrovascular diseases. For example, borneol is capable of passing through cardiocerebral barrier, while propanoid acid is not prone to pass through cardiocerebral barrier. Therefore, propanoid acid may be modified in structure by incorporating the chemical structure of borneol.

### Disclosure of the Invention

One object of the present invention is to provide a substituted β-phenyl-α-hydroxy-propionic acid derivative and a process for synthesizing the same, as well as use of the substituted β-phenyl-α-hydroxy-propionic acid derivative in the manufacture of a medicament for prevention and treatment of cardiovascular and cerebrovascular diseases.

In one aspect of the present invention a substituted β-phenyl-α-hydroxy-propionic acid derivative, specifically a compound of the formula (I), is provided:

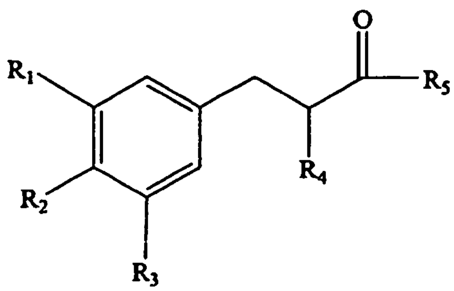

**(I)**

wherein $R_1$ and $R_2$ respectively are OH, $R_3$=H, $R_4$=OH, and $R_5$=

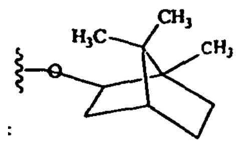

,

i.e., the compound is bornyl β-(3,4-dihydroxylphenyl)-α-hydroxylpropionate ester as shown in formula (II).

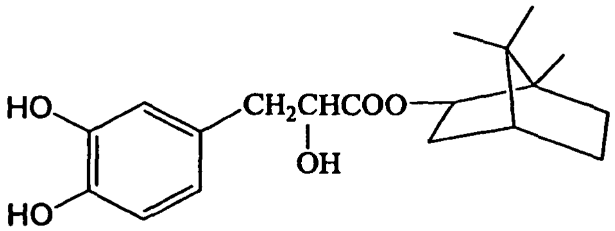

(II)

In another aspect of the present invention, a process for synthesizing a compound of the formula (I), is provided, which comprises: reacting a compound of the formula (III) with a compound of formula (IV) or a hydrate thereof in the presence of a catalyst:

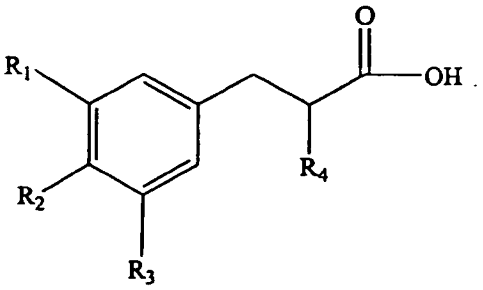

$R_5$-H

(III) (IV)

Wherein $R_1$, $R_2$, $R_3$, $R_4$ and $R_5$ have the same meanings as defined above for the formula (I).

The said catalyst can be selected from concentrated $H_2SO_4$, silicotungstic acid, phosphomolybdic acid, p-toluene sulfonic acid, $S_2O_8^{2-}/ZrO_2$, aluminum trichloride, zinc chloride and/or magnesium chloride. Preferably, the said catalyst is p-toluene sulfonic acid, $S_2O_8^{2-}/ZrO_2$, aluminum trichloride and/or zinc chloride. It is particularly advantageous to use p-toluene sulfonic acid and/or $S_2O_8^{2-}/ZrO_2$.

The reaction molar ratio of the compound of the formula (III) to the compound of the formula (IV) can be 1: 0.8~1:1.5, preferably 1:1~1:1.5, more preferably 1:1.25~1:1.5, most preferably 1:1.5.

Optionally, the reaction is conducted in a solvent. The solvent may be selected from the group consisting of ethyl acetate, dichloromethane, tetrahydrofuran, acetone, toluene, 1,4-dioxane and *N,N*-dimethylformamide alone or in any combination. Preferably, the solvent is selected from the group consisting of tetrahydrofuran, acetone, toluene, 1,4-dioxane, and *N,N*-dimethylformamide. More preferably, the solvent is selected from tetrahydrofuran and acetone. Most preferably tetrahydrofuran is used as the solvent. These solvents can be used either alone or in any combination.

The reaction temperature can be varied depending on the solvent being used. Advantageously, it is controlled within the range of 0°C~150°C. Preferably, the reaction temperature is 25°C~100°C. More preferably , the reaction temperature is 65°C.

The reaction duration can be 2h~24h, preferably 5h~15h, more preferably 8h~12h, most preferably 8h.

In one specific embodiment, there provided is a process for synthesizing a compound of the formula (II), which comprises: reacting β-(3,4-dihydroxylphenyl)-α-hydroxylpropionic acid with borneol in the presence of a catalyst. The catalyst can be a Lewis acid catalyst, such as toluene sulfonic acid, $S_2O_8^{2-}/ZrO_2$, aluminum trichloride and/or zinc chloride, preferably $S_2O_8^{2-}/ZrO_2$. In the said process, the molar ratio of β-(3,4-dihydroxylphenyl)-α-hydroxylpropionic acid to borneol can be 1:1~1:1.5, preferably 1:1.25~1:1.5, more preferably 1:1.5. The reaction is conducted in a solvent, which can be selected from the group consisting of tetrahydrofuran, toluene, 1,4-dioxane or *N,N*-dimethylformamide, preferably tetrahydrofuran. The reaction temperature can be varied depending on the solvent being used, and is generally controled within the range of 65°C~150°C, preferably at 65°C. The reaction duration can be 8h~12h, preferably 8h.

When $S_2O_8^{2-}/ZrO_2$ is used as the catalyst, the $S_2O_8^{2-}/ZrO_2$ can be optionally prepared by the following process: adding aqueous ammonia to $ZrOCl_2$ solution at 0~10°C to reach a pH of 9-12, aging, washing the pellets to be free of $Cl^-$, heating in an oven to dryness, grinding, then adding into a solution of $(NH_4)_2S_2O_8$ for dipping, filtrating, drying, grinding, and then baking at 500~700°C for 2-5h, resulting in $S_2O_8^{2-}/ZrO_2$.

In a further aspect of the present invention, there is provided the compound of the present invention for use in the prevention and treatment of cardiovascular or cerebrovascular diseases.

Brief Description of the Drawings

Fig. 1 shows a scheme for the synthesis of the compound of the formula (II) in Example 1, i.e., bornyl β-(3,4-dihydroxylphenyl)-α-hydroxylpropionate ester.

Fig.2 shows the mass spectrum of the final product obtained in Example 1.

Fig.3 shows the infrared spectrum of the final product obtained in Example 1.

Fig.4 shows the $^1$HNMR spectrum of the final product obtained in Example 1.

Fig.5 shows the $^{13}$CNMR spectrum of the final product obtained in Example 1.

The present invention is further illustrated in conjunction with the following examples on synthesis and the pharmacodynamic tests. However, it should be understood that these examples are merely intended to illustrate, but by no way to limit, the present invention.

Example 1: Synthesis (I) of bornyl β-(3,4-dihydroxylphenyl)-α-hydroxylpropionate ester

(1) Synthesis of acetyl glycine

Into a 250mL three-necked, flask, 0.33mol glycine and 100mL destilled water were added and vigorously agitated until dissolution, and 0.67mol acetic anhydride was slowly added dropwise under stirring. The mixture was vigorously agitated continuously for 50 min, then sucking filtered. The pellets were washed and dried, resulting in a white crystal with a yield of 86.0%.

(2) Synthesis of 2-methyl-4-(3,4-diacetoxybenzylidene)-oxazolone

Into a 250mL three-necked flask, 0.20mol 3,4-dihydroxylbenzaldehyde, 0.24mol acetylglycine and 0.26mol anhydrous sodium acetate were added, then 189mL acetic anhydride was added, and mixed homogeneously by stirring. The reaction was performed for 4h in a 80°C. water bath under stirring, then the temperature was elevated to 100°C and the reaction was continued for 1h under stirring. The reaction mixture was cooled to room temperature and then placed in a refrigerator for further cooling. 100mL water was added to the reaction mixture under stirring to precipitate a yellow crystal at the bottom. After sucking filtration, washing and drying, a yellow crystal was obtained in a yield of 75.0%.

(3) Synthesis of β-(3,4-diacetoxyphenyl)-α-acetamidoacrylic acid

Into a flask, 0.15mol 2-methyl-4-(3,4-diacetoxybenzylidene) oxazolone, 166mL acetone and 166mL distilled water were added, then slowly heated to boiling, and refluxed by heating for 3h. The mixture was decolorized using active carbon. After filtration, the filtrate was placed for crystallization, then sucking-filtered, washed and dried resulting in an ecru crystalline powder in a yield of 72.9% was obtained.

(4) Synthesis of β-(3,4-dihydroxylphenyl)pyruvic acid

Into 0.25mol β-(3,4-diacetoxyphenyl)-α-acetamidoacrylic acid, 1500mL of 1mol·$L^{-1}$ hydrochloric acid was added. Then the mixture was heated for reflux under stirring for 8h. After decolorization using active carbon and sucking filtration, the filtrate was concentrated to precipitate as a crystal. The mixture was sucking-filtered, washed and dried, resulting in a white loose crystal in a yield of 48.1%.

(5) Synthesis of β-(3,4-dihydroxylphenyl)-α-hydroxylpropionic acid

Into 0.17mol β-(3,4-dihydroxylphenyl) pyruvic acid, 112g zinc amalgam and 1808mL of 1.4 mol·$L^{-1}$ hydrochloric acid solution were added, and the reaction was performed under heating and refluxing for 8h. After filtration, the filtrate was extracted using ethyl acetate repeatedly, dried using anhydrous $Na_2SO_4$. After removal of ethyl acetate, β-(3,4-dihydroxylphenyl)-α-hydroxylpropionic acid was obtained in a yield of 40.3%.

(6) Synthesis of bornyl β-(3,4-dihydroxylphenyl) -α-hydroxylpropionate ester

Into a flask, 0.12mol β-(3,4-dihydroxylphenyl)-α-hydroxylpropionic acid and 0.18mol borneol were added, then 0.86g p-toluene sulfonic acid or 2.00g of self-made $S_2O_8^{2-}/ZrO_2$ was added as catalyst, and 500mL tetrahydrofuran was added. The reaction was performed at 65°C for 8h. After the completion of the reaction, the catalyst, the solvent and the unreacted borneol were removed to obtain a brown viscous substance, which was further separated using column chromatography to obtain a yellowish oil.

The catalyst $S_2O_8^{2-}/ZrO_2$ was prepared by a process comprising the following steps: preparing a solution of 1mol·$L^{-1}$ $ZrOCl_2$ with 0.025mol $ZrOCl_2 \cdot 8H_2O$; stirring in an ice-water bath; adding slowly 6 mol·$L^{-1}$ aqueous ammonia dropwise until pH reached 10; aging for 12h. suction filtrating; washing the filter cake with distilled water until no $Cl^-$ could be determined (using 0.1mol·$L^{-1}$$AgNO_3$ test); baking the filter cake at 110°C for 10h; comminuting; soaking in 0.5mol·$L^{-1}$ $(NH_4)_2S_2O_8$ solution for 12h; suction filtrating; drying; comminuting; and baking in a muffle furnace at 600°C for 3h to obtain $S_2O_8^{2-}/ZrO_2$.

(7) The mass spectrum, infrared spectrum, $^1$HNMR spectrum and $^{13}$CNMR spectrum of the resulting yellowish oil

Fig.2 is the mass spectrum of the obtained yellowish oil, which shows 351.7 is the molecular ion peak of ($M+H_2O$), and the oil has a molecular weight of 333.69;

Fig.3 shows the IR(KBr) v/$cm^{-1}$: 3363.61(OH), 2953.12($CH_3$), 2913.90($CH_2$), 1725.51 (C=O), 1608.20, 1521.53, 1450.32(backbone of the benzene ring ), 1281.36(C=O of the ester), 1114.39(C-O of the secondary hydroxyl), 885.71 and 805.68(1,2,4-trisubstituted benzene ring);

Fig.4 shows the $^1$HNMR($CD_3COCD_3$, 500MHz)δ: 6.57-7.64(m, 3H, Ar-H), 4.10-4.32(m, 1H, -CH(OH)-), 4.83(t, 1H, -CH-), 2.79-2.92(m, 2H, $-CH_2-$);

Fig.5 shows the $^{13}$CNMR($CDCl_3$, 500MHz)δ: 174.790, 143.807, 143.056, 128.557, 121.549, 116.895, 115.488, 81.983, 71.646, 48.860, 47.881, 44.798, 39.871, 36.506, 27.918, 27.057, 19.653, 18.774, 13.501.

The above characterization data proved that bornyl β-(3,4-dihydroxylphenyl)-α-hydroxylpropionate ester was obtained by synthesis.

Example 2: Synthesis (II) of bornyl β-(3,4-dihydroxylphenyl)-α-hydroxylpropionate ester

The synthesis was performed through the same procedure of Example 1, except that 0.12mol β-(3,4-dihydroxylphenyl)-α-hydroxylpropionic acid and 0.15mol borneol were added to the three-necked flask, then 0.86g p-toluene sulfonic acid as catalyst and 500mL tetrahydrofuran were added, and the reaction was performed at 65°C for 12h. After the completion of the reaction, the reaction solvent was removed by vacuum distillation, and the resulting viscous substance was treated by vacuum ($1.3\times10^{-3}$Pa) using an oil pump in a boiling-water bath to remove borneol, then 200mL ethyl acetate was added. The resulting solution was washed with saturated $NaHCO_3$ solution to remove unreacted β-(3,4-dihydroxylphenyl) -α-hydroxylpropionic acid and p-toluene sulfonic acid. The obtained ethyl acetate layer was concentrated under reduced pressure, resulting in a brown viscous substance, which was further separated by column chromatography, obtaining a yellowish oil. The resultant yellowish oil has the same mass spectrum and infrared spectrum as that in Example 1.

Example 3: Synthesis (III) of bornyl β-(3,4-dihydroxylphenyl) -α-hydroxylpropionate ester

The synthesis was performed through the same procedure of Example 1, except that 0.1 mol β-(3,4-dihydroxylphenyl)-α-hydroxylpropionic acid and 0.12mol borneol were added to the three-necked flask, then 1.33g $S_2O_8^{2-}/ZrO_2$ as catalyst and 400mL 1,4-dioxane were added, and the reaction was performed at 100°C for 8h. After the completion of the reaction, the catalyst $S_2O_8^{2-}/ZrO_2$ was removed by sucking filtration, and the solvent was removed by vacuum distillation, and the resulting viscous substance was treated by vacuum ($1.3\times10^{-3}$Pa) using an oil pump in a boiling-water bath to remove borneol. The resultant black brown viscous substance was separated using column chromatography, obtaining a yellowish oil, which has the same mass spectrum and infrared spectrum as that in Example 1.

Example 4: Synthesis (IV) of bornyl β-(3,4-dihydroxylphenyl)-α-hydroxylpropionate ester

The synthesis was performed through the same procedure of Example 1, except that 0.06mol β-(3,4-dihydroxylphenyl)-α-hydroxylpropionic acid and 0.09mol borneol were added to the three-necked flask, then 0.60g aluminum trichloride as catalyst and 200mL *N,N*-dimethylformamide as solvent were added, and the reaction was performed at 150°C for 10h. After the completion of the reaction, the solvent was removed by vacuum distillation, and the resulting viscous substance was treated by vacuum ($1.3\times10^{-3}$Pa) using an oil pump in a boiling-water bath to remove borneol. The resultant black brown substance was separated using column chromatography, obtaining a yellowish oil, which has the same mass spectrum and infrared spectrum as that of Example 1.

Example 5: Pharmacodynamic test

1. Effects of bornyl β-(3,4-dihydroxylphenyl) -α-hydroxylpropionate ester (hereinafter briefly cited as "bornyl salvianate ester") on blood flow of cerebral microcirculation in rats with middle cerebral artery occlusion

60 SD rats with a body weight of 220±20g were randomly divided into normal control group, model contral group, salvianic acid injection group (ip. 1mL/kg), and group of bornyl β-(3,4-dihydroxylphenyl)- α-hydroxylpropionate ester at a small, middle and large dose respectively (ip. 5, 15, and 35mg/kg). The rats of normal group and model group were administered with equal-volume of physiologic saline by ip. The rats were anesthetized by intraperitoneal injection of 1% pentobarbital sodium at an amount of 40mg/kg, then placed at supine position, head fixed, and cut skin along cervical middle line. Trachea cannula, was inerted into the rats and they were allowed to spontaneous respire. Right common jugular vein and common carotid artery were isolated and a suture was introduced for later use. Animals were fixed on a rat stereotaxic apparatus, a cranial window with a size of 6x8mm was opened at the right temple with a dental drill, after hemostasis, cerebral dura mater was cut open with scissors to expose cerebral pia mater. The window was covered and sealed with glass and dental cement, and the laser probe of a laser-doppler microcirculatory blood flow meter was fixed on the cranial window. Then the animals were fixed at lateral position, the right common carotid artery was lifted, ligated at the proximal part and carefully cut with scissors, a nylon suture having a diameter of about 0.3mm was introduced into the artery. The distance between the artery cut and the paropia of the rats was marked before the suture was introduced. When the nylon suture was introduced to approach the marked position, the speed for introducing the suture should slow down and the cerebral microcirculatory blood flow exhibited by the laser-doppler microcirculatory blood flow meter should be observed simultaneously. When the suture arrived at middle cerebral artery, an abrupt decrease of the microcirculatory blood flow would be observed. After the observation of a decrease in the microcirculatory blood flow, the suture was further penetrated by about 1 mm, and the distal end of the cut and the suture in the artery were ligated securely. Spare suture was cut-off. After the end of the test, it was checked whether the nylon suture blocked the initiation site of the middle cerebral artery, and the data of any animal whose artery was not blocked were cancelled. The animals of the control group were not treated. After the cranial window was prepared, the probe of J I2200 type laser-doppler microcirculatory blood flow meter was fixed at the cranial window, and the probe was maintained without displacement and rotation during the whole test. The microcirculatory blood flows before ligation and 5, 15, 30, 45 and 60min after ligation were recorded, and the data of the animals in the drug-treatment groups were recorded at the same time points. The mean microcirculatory blood flow observed within 1 min at each time point was designated as the microcirculatory blood flow of the respective time point.

Table 1. Effects on cerebral microcirculatory blood flow in rats with middle cerebral artery occlusion (n=10)

| Group | Dose | Microcirculatory blood flow (mL) | | | | |
|---|---|---|---|---|---|---|
| | | 5min | 15min | 30min | 45min | 60min |
| Control | / | 65.3±5.7 | 67.4±7.4 | 67.8±6.8 | 65.1±5.9 | 64.2±8.5 |
| Model | / | 10.2±2.3 | 11.4±3.4 | 13.2±4.1 | 14.0±3.4 | 14.9±6.5 |
| Salvianic acid | 1mL/kg | 10.8±3.6 | 20.1±6.9** | 31.2±9.5** | 33.0±6.8** | 29.4±6.9** |
| Test Drug | 5mg/kg | 11.4±5.4 | 12.4±4.6 | 12.9±5.1 | 16.1±4.1 | 16.0±6.9 |
| Test Drug | 15mg/kg | 9.4±3.7 | 12.8±5.6 | 20.7±3.2* | 25.0±5.2** | 24.4±4.8** |
| Test Drug | 35mg/kg | 10.8±2.7 | 19.4±5.1** | 32.7±6.2** | 35.4±8.2** | 35.2±5.8** |

In comparison with the model group: *$P$<0.05, **P<0.01

The test results showed that, after the middle cerebral artery was blocked, the cerebral microcirculatory blood flow in blood-supply region (frontal and parietal lobe) decreased rapidly and kept at a relatively low level. Only after the artery was blocked for 30min did the cerebral microcirculatory blood flow increase slightly, which indicated that cerebral ischemia models were successfully established. Meanwhile the cerebral microcirculatory blood flow increased slightly 30 minutes or 15 minutes after the administration of bornyl β-(3,4-dihydroxylphenyl)- α-hydroxylpropionate ester at 15mg/kg and 35mg/kg respectively. These results suggest the ditation of arteriole, and the increase of microcirculatory blood flow may have possible positive effects on ischemic cerebrovascular diseases, but the corresponding action mechanism needs to be investigated.

2. Protection effects of bornyl salvianate ester on cardiac ischemia reperfusion (I/R) injury

52 SD rats having a body weight of 220±20g were randomly divided into model control group, salviannic acid injection group (im 1mL/kg), and group of bornyl β-(3,4-dihydroxylphenyl)-α-hydroxylpropionate ester at a small, middle and large dose (ip 5, 15, 35mg/kg). The rats of the normal group and the model control group were ip administered with equal-volume of physiologic saline. The rats of all groups were administered for consecutive 5 days. When the rats were administered for the last time, they were simultaneously anesthetized with 1.5% pentobarbital sodium (ip 45mg/kg), then a catheter was inserted into the right carotid artery, and connected via a transducer to an eight-edchanneled physiological recorder. Tracheal cannula was performed, and the ventilation rate was maintained at 60 times/min. The chest was opened, a 6/0 suture was used to form a loop at a site 1~2mm from the root of the anterior descending coronary artery, and a plastic pipe was introduced through the loop, then the loop was tightened up. The change of electrocardiogram was observed. An increase or decrease of ST indicated the success of ligation. The color of the myocardial tissue below the ligation suture became darker. 30 minutes later, the plastic pipe was pulled out to allow coronary artery blood flowing again and cause hyperemia of local tissue during reperfusion. For the groups subjected to ischemia for 30min and reperfusion for 30min, the myocardial infarction areas were recorded before the test, after ischemia for 1min and 30min, and after reperfusion for 30min; for the groups subjected to ischemia for 30min and reperfusion for 2h, cardiac tissue samples were taken and fixed using 10% formalin, embedded with paraffin, serially sliced into sections with a thickness of 4μm, and separately subjected to immunohistochemistry tests; the rats of the sham operation group were subjected only to the introduction of suture but their coronary arteries were not ligated.

Effects on myocardial infarct areas caused by cardiac muscle I/R

After the rats were subjected to ischemia and reperfusion for 30minutes, their anterior descending coronaries were ligated again, then they were sacrificed, their hearts were removed promptly, and 0.5mL of 1% evans blue was injected into heart chambers via aorta to distinguish the ischemic regions and the non-ischemic regions. After the heart atrium and right ventricle were cut-off, the heart was refrigerated at -20°C for 30min, then placed in a tailor-made slice groove, and cut along the long axis to form 2mm slices. The slices were immersed in 1% TTC phosphoric acid buffer solution (pH7.4), and incubated at 37°C for 30min to distinguish risky regions and necrotic regions. Then the slices were fixed with 10% formaldehyde for 24h to enhance staining color for contrast photography. After the above treatment, the myocardial tissue was divided into: normal myocardia in blue, ischemic myocardia in light red, and necrotic myocardion in gray. A computer image analysis software was used to calculate the percentage of the area of the infarct myocardial region (nec) based on the area of the risky myocardial region (aar, i.e., ischemic myocardia, including ischemic infarct region and ischemic non-infarct region)(nec/aar), and the percentage of the area of the infarct myocardial region based on the whole area of the myocardia(nec/lv) to indicate the degree of infarct, as well as the percentage of the area of the

risky myocardia based on the area of the left ventricle(aar/lv).

Table 2. Effects on myocardial infarction areas caused by cardiac muscle I/R

| Group | Dose | Animal Number | aar/lv(%) | nec/lv(%) | nec/aar(%) |
|---|---|---|---|---|---|
| Model control | / | 12 | 67.10±11.40 | 50.52±15.65 | 64.10±13.03 |
| Salvianic acid | 1mL/kg | 10 | 50.21±7.10* | 41.41±5.49* | 49.87±7.83* |
| Test drug | 5 mg/kg | 9 | 64.09±18.10 | 48.45±17.11 | 61.72±14.65 |
| Test drug | 15mg/kg | 10 | 63.02±15.98 | 48.23±15.34 | 62.63±13.82 |
| Test drug | 35 mg/kg | 11 | 51.97±13.04 * | 40.21±12.65 * | 50.01±9.03* |

In comparison with the model control group: *$P$ < 0.05

The results showed that, in comparison with the model control group, the values of aar/lv, nec/lv and nec/aar in the large dose group respectively decreased by 22.5%, 20.4% and 22% (P<0. 01), suggesting that the area of the myocardial infarct caused by myocardia I/R can be lowered.

Effects on the protein expression of Bax, Bcl-2, caspase-3, MMP-2 and PPARγ

Standard immunohistochemistry ABC and SP methods were employed for staining. Bax: anti-rabbit polyclonal antibody (Santa CruzBio. Inc.) at a dilution of 1:200; Bcl22: anti-rabbit polyclonal antibody (TBD Tianjin Biotechnological Center). At a dilution of 1100; caspase-3: anti-rabbit polyclonal antibody (Normarkers Fromont, CA), at a dilution of 1: 200; MMP-2: anti-murine monoclonal antibody (NormarkersFromont, CA) at a dilution of 1:200; PPARγ: anti-caprine polyclonal antibody (Santa Cruz Bio. Inc.), at a dilution of 1:500. The specific procedures were performed in accordance with the instructions of the ABC and SP kits, DAB was used for color development, and neutral resin was used for mounting. PBS was used instead of the first antibody as a negative control. Cells with positive expression of the test polypeptides were of brown-yellow color, with MMP-2 protein being present in acytoplasm, Bcl-2 expressed in nuclear membrane and cytoplasm, Bax mainly in cytoplasm and partially in nucleus, and caspase-3 mainly in nucleus and partially in cytoplasm. CMIAS image analysis system was used for random selection of fields of slices and automatic selection of sites for analysis, and statistic analysis was performed by using mean optical density values or integral optical density values of the obtained myocardial tissue slices.

Table 3. Effects on the protein expression of Bax, Bcl-2, caspase-3, MMP-2, and PPARγ (n=10)

| Group | Dose | OD value | | | | |
|---|---|---|---|---|---|---|
| | | Bax | Bcl-2 | Caspase-3 | MMP-2 | PPARγ |
| Model control | / | 0.13±0.04 | 0.14±0.03 | 0.37±0.11 | 0.16±0.04 | 0.17±0.02 |
| Salvianic acid | 1mL/kg | 0.08±0.02* | 0.18±0.04 | 0.21±0.12** | 0.12±0.05 | 0.34±0.08** |
| Test drug | 5 mg/kg | 0.12±0.03 | 0.15±0.03 | 0.35±0.14 | 0.14±0.05 | 0.16±0.05 |
| Test drug | 15 mg/kg | 0.09±0.05 | 0.15±0.06 | 0.32f0.08 | 0.14±0.03 | 0.18±0.06 |
| Test drug | 35mg/kg | 0.07±0.03* | 0.21±0.04* | 0.20±0.07** | 0.11±0.03* | 0.18±0.10 |

In comparison with the model control group: *$P$ < 0. 05, **$P$ < 0. 01

The results showed that the expression of Bcl-2 and Bax was indeed changed in the myocardial cells I/R, indicating that they participate in the regulation of cell apoptosis. Bornyl β-(3,4-dihydroxylphenyl)-α-hydroxylpropionate ester could reduce the expression of Bax and caspase-3 proteins and increase the expression of Bcl-2 protein, suggesting that bornyl β-(3,4-dihydroxylphenyl)-α-hydroxylpropionate ester could initiate the self-protective mechanism of cells against injury by inducing the expression of Bcl-2 and reducing the levels of Bax and caspase-3, reverse the cell apoptosis and necrosis processed stimulated by the myocardiac I/R, thereby exhibit protection effects on myocardial cells.

MMP-2 is associated with cardiac muscle I/R injury, which is achieved by the cleavage of troponin I, which in turn would directly lead to cell apoptosis. MMP-2 specific inhibitor could improve the cardiac function of rats with myocardiac I/R, and the test results indicated that bornyl β-(3,4-dihydroxylphenyl)-α-hydroxylpropionate ester could cause the decrease of MMP-2 protein, which could be another mechanism for bornyl β-(3,4-dihydroxylphenyl)- α-hydroxylpropionate ester to protect myocardia from I/R injury.

3. Effects of bornyl salvianate ester on blood pressure and left ventricular function of anesthetized rats

Rats were anesthetized by intraperitoneal injection of 20% urethane 5mL/kg and fixed; the skin of neck of rats were incised, anterior cervical muscles were isolated, trachea was exposed, and tracheal cannula was inserted; common carotid artery was isolated, a cardiac catheter was introduced through the common carotid artery into left ventricle, the left intraventricular pressure was measured with a pressure transducer (T-200) of RM-6000 multi-channel polygraph and the carrier amplifier (AP-601G) of RM-6000 multi-channel polygraph, then the signals of the left intraventricular pressure were input to the differential amplifier (ED-601G) of RM-6000 multi-channel polygraph to record the maximum rate of increasing and decreasing the left intraventricular pressure ($dp/dt_{max}$ $-dp/dt_{max}$); right femoral artery was isolated, the arterial blood pressure was measured by using cannula; a recording electrode of electrocardiograph was connected to record type II electrocardiogram. All data were input into PowerLab/8Sp data acquisition and processing system via RM-6000 multi-channel polygraph, and recorded, analyzed and processed by PowerLab/8Sp.

The abdomen was opended 1.5cm below xiphoid bone to isolate duodenum, a small incision was formed on the duodenum away from blood vessels by ophthalmic scissors, a catheter was inserted and the incision was fixed by suture for administration. After the end of operation and a further waiting period of 30min, normal data were recorded once the indexes to be monitored were stable.

Test drugs were duodenally administered via the cathether, and the indexes were monitored 5, 15, 30, 60, 90 and 120min after the administration. The change ratios of the indexes were calculated in accordance with the following formula, and were used for statistic analysis among groups.

$$Change_ratio(\%) = \frac{(After_administration - Before_administration)}{Before_administration} \times 100$$

3.1 Effects on the heart rate of anesthetized rats

The test results indicated that the 4.5mg/kg, 9mg/kg and 18mg/kg doses of bornyl salvianate ester showed no significant effects on the heart rate of anesthetized rats, no significant difference can be observed as compared to the blank control group; while verapamil hydrochloride significantly reduced the heart rate and significant difference can be observed as compared to the model group ($P<0.05$ or $P<0.01$) (Table 4).

3.2 Effects on mean arterial pressure, systolic pressure and diastolic pressure of anesthetized rats

In the group at the does of 18mg/kg bornyl salvianate ester, the mean arterial pressure, systolic pressure and diastolic pressure of anesthetized rats significantly decreased after administration, and significant differences ($P<0.05$ or $P<0.01$) as compared to the blank control group were observed at the timepoints of 15, 60, 90 and 120 min; in the 9mg/kg group of bornyl salvianate ester, the mean arterial pressure, systolicsystolic pressure and diastolic pressure of anesthetized rats exhibited a decrease tendency, and significant differences ($P<0.05$ or $P<0.01$) from the blank control group were observed at 15 and 60 min; in the 4.5mg/kg group of bornyl salvianate ester, the mean arterial pressure, systolicsystolic pressure and diastolic pressure of anesthetized rats exhibited no significant change, and no significant difference from the blank control group were observed; while verapamil hydrochloride could significantly reduce the mean arterial pressure, systolicsystolic pressure and diastolic pressure of anesthetized rats, and significant differences ($P<0.01$) from the model control group were observed at 5, 15, 30, 60, 90 and 120 min (Tables 5, 6 and 7).

3.3 Effects on left intraventricular pressure of anesthetized rats

In the 18mg/kg group of bornyl salvianate ester, the left intraventricular pressure of anesthetized rats significantly decreased after administration, and significant differences ($P<0.05$ or $P<0.01$) from the blank control group were observed at 15, 30, 60, 90 and 120 min; in the 9mg/kg group of bornyl salvianate ester, the left intraventricular pressure of anesthetized rats exhibited a decrease tendency, and significant differences ($P<0.05$ or $P<0.01$) from the blank control group were observed at 60 min; in the 4.5mg/kg group of bornyl salvianate ester, the left intraventricular pressure of anesthetized rats was not significantly affected after administration, and no significant difference from the blank control group was observed; while verapamil hydrochloride significantly reduced the left intraventricular pressure of anesthetized rats, and significant differences ($P<0.01$) from the model control group were observed (Table 8).

3.4 Effects on dp/dt and -dp/dt of anesthetized rats

In the 18mg/kg group of bornyl salvianate ester, the the dp/dt of anesthetized rats significantly decreased after administration, and significant differences ($P<0.05$ or $P<0.01$) from the blank control group were observed at 15, 30, 60, 90 and 120 min; in the 9mg/kg group of bornyl salvianate ester, the dp/dt of anesthetized rats exhibited a decrease tendency, and significant differences ($P<0.05$ or $P<0.01$) from the blank control group were observed at 60 and 120min; in the 4.5mg/kg group of bornyl salvianate ester, the dp/dt of anesthetized rats was not significantly affected after administration, and no significant difference from the blank control group was observed; while verapamil hydrochloride significantly reduced the dp/dt of anesthetized rats, and significant differences ($P<0.01$) from the model control group were observed.

In the 9mg/kg and 18mg/kg groups of bornyl salvianate ester, the -dp/dt of anesthetized rats exhibited a decrease tendency, and significant differences ($P<0.05$ or $P<0.01$) from the blank control group were observed at 60 and 120min; in the 4.5mg/kg group of bornyl salvianate ester, the -dp/dt of anesthetized rats was not significantly affected after administration, and no significant difference from the blank control group was observed; while verapamil hydrochloride significantly reduced the -dp/dt of anesthetized rats, and significant differences ($P<0.01$) from the model control group were observed (Tables 9, 10).

The test results indicate that bornyl salvianate ester could reduce left intraventricular pressure, dp/dt and -dp/dt, which suggests that bornyl salvianate ester has effects of reducing the negative cardiac efficiency of myocardial contractility, and the effects on negative cardiac efficiency could be the reason of the decrease of the mean arterial pressure, systolic pressure and diastolic pressure of anesthetized rats.

In the meantime, the test results showed that the $dp/dt_{MAX}$ decreased, but the heart rate did not change significantly, i.e., the $dp/dt_{MAX}$ was not in direct correlation with the heart rate. The mechanism for these phenomena needs further studying.

4. Protective effects of bornyl salvianate ester on acute myocardial ischemia in rats

60 Male rats were randomly divided into sham operation group (0.5% Poloxamer, 10mL/kg), model control group (0.5% Poloxamer, 10mL/kg), verapamil group (verapamil tablet, 10mg/kg), and bornyl salvianate ester groups (10mg/kg, 20mg/kg and 40mg/kg). At 0.5h after the rats of these groups were administered intragastrically, the standard II lead electrocardiogram (before modeling) was recorded, and the height of ST-T segment was measured. The rats of sham operation group were then subjected to coronary artery braid without ligation , and the rats of other groups were subjected to coronary artery ligation in accordance with the following method to establish cute myocardial ischemia models. The rats were subjected to etherization, fixed at supine position, and their normal electrocardiograms (before modeling) were recorded. The skin of left thorax was incised under aseptic conditions, the $4^{th}$ intercostal muscles were isolated in blunt way, the heart was extruded by light pressure to the right thorax, the left anterior descending coronary artery was ligated at a position 2~3mm departing from the left coronary artery origin and between the pulmonary conus and the left auricle of heart, then the heart was sent back to thorax cavity immediately and sutured the incision. Penicillin was smeared topically for the prevention of infections. After operation, the electrocardiogram of postischemia was recorded immediately (at 0 min after modeling), and the height of ST-T segment was measured. At 24h after the animals of these groups underwent the operation, they were anesthetized by peritoneal injection of 20% urethane 5mL/kg, and the electrocardiogram was recorded again (at 24h after modeling), and the height of ST-T segment was measured; blood samples were drawn from abdominal aorta, sera were separated, and the activities of lactate dehydrogenase (LDH), creatine kinase (CK), creatine kinase isoenzyme (CK-MB) and superoxide dismutase (SOD) and the content of malondialdehyde (MDA) were measured; the heart was taken out by open chest and washed with cold physiological saline, the heart atrium was removed, the heart ventricle was cross cut to form 3~4 slices, the slices were dipped in 0.25% NBT solution and stained under 37°C water-bath for 10min, the infarct cardiac muscles were cut and weighed, and the weight percentage of the infarct cardiac muscles based on the cardiac muscles of the whole heart ventricle was calculated (Tables 11-14).

The results showed that in all the bornyl-salvianate-ester dosed groups, the proportion of the infarct cardiac muscles in the whole ventricle was reduced, in which relative potent effects were observed in the middle and large dose groups ($P<0.05$ or $P<0.01$); in all dose groups, the activities of lactate dehydrogenase (LDH), creatine kinase (CK), creatine kinase isoenzyme (CK-MB) decreased 24h later; the superoxide dismutase (SOD) in rats with acute myocardial ischemia increased, especially in the large dose group ($P<0.05$); in middle and large dose groups, a decrease tendency of the serum MDA content was observed, but no significant difference from the model control group was observed; at 24h after coronary artery ligation, the ST-T segment elevation in the electrocardiogram was observed, especially in the large dose group ($P<0.05$). The results indicated that the bornyl salvianate ester could reduce the area of myocardial infarction in rats, and exhibit protective effects on acute myocardial ischemia in rats.

Table 4. Effects on heart rate (beat/min)

| Group | Dose | Before administration | After administration | | | | | |
|---|---|---|---|---|---|---|---|---|
| | | | 5' | 15' | 30' | 60' | 90' | 120' |
| Blankcontrol (n=10) | 2.5mL/kg | 344.39±42.73 | 344.28±46.02<br>2.23±5.30 | 355.3±44.09<br>5.66±7.27 | 349.22±47.61<br>4.44±10.34 | 354.23±47.61<br>6.18±11.78 | 361.39±48.94<br>8.06±15.53 | 359.78±54.27<br>7.37±15.32 |
| Tween control (n=10) | 2.5mL/kg | 322.67±58.28 | 326.83±50.06<br>2.42±11.78 | 38S.47±46.72<br>4.05±20.61 | 33791±49.80<br>6.63±18.47 | 332.98±55.18<br>5.03±19.64 | 333.84±54.39<br>5.46±19.62 | 327.03±57.06<br>3.30±20.73 |
| Propanoid acid borneol ester (n=10) | 18mg/kg | 357.28±36.29 | 358.56±44.88<br>0.22±4.94 | 356.00±40.82<br>-0.07±9.93 | 348.07±39.21<br>-2.01±12.45 | 357.22±47.10<br>0.39±13.05 | 355.33±9.61<br>-0.44±691 | 357.68±53.26<br>0.28±12.04 |
| Propanoid acid borneol ester (n=10) | 9mg/kg | 373.43±41.28 | 374.04±47.60<br>0.28±7.83 | 374.93±47.51<br>0.48±7.36 | 361.94±43.56<br>-3.00±5.83 | 359.61±39.30<br>-4.52±7.13 | 35438±62.30<br>-4.13±17.94 | 354.09±47.98<br>-4.62±12.34 |
| Propanoid acid borneol ester (n=10) | 4.5mg/kg | 329.43±43.77 | 337.41±47.67<br>2.49±7.18 | 347.56±17.18<br>5.67±7.58 | 348.9±52.11<br>5.97±9.72 | 365.43±32.77<br>12.05±12.32 | 369.71±35.54<br>13.31±13.05 | 360.36±51.93<br>9.40±6.30 |
| Verapamil control (n=10) | 11.4mg/kg | 363.19±46.67 | 345.03±33.81<br>-4.63±4.08 | 32496±36.20<br>-9.67±11.64" | 322.02±51.09<br>-10.32±16.56' | 317.91±5804<br>-11.31±19.30' | 329.61±55.70<br>-7.75±20.11 | 328.05±58.98<br>-7.96±21.39 |

In comparison with the Tween control group: *p< 0.05, **p<0.01

Table 5. Effects on contractive pressure (mmHg)

| Group | Dose | Before administration | After administration | | | | | |
|---|---|---|---|---|---|---|---|---|
| | | | 5’ | 15’ | 30’ | 60’ | 90’ | 120’ |
| Blankcontrol (n=10) | 2.5mL/kg | 117.57±21.61 | 121.76±23.27<br>3.55±5.90 | 123.75±26.54<br>498±9.25 | 11891±32.99<br>-0.09±15.39 | 118.80±31.50<br>0.07±10.36 | 118.40±33.16<br>-0.17±12.98 | 124.51±35.18<br>5.80±24.03 |
| Tween control (n=10) | 2.5mL/kg | 124.41±26.13 | 122.64±26.97<br>-1.33±6.67 | 124.75±24.75<br>1.08±9.91 | 121.92±23.44<br>-0.79±13.70 | 120.80±29.92<br>2.46±16.70 | 121.05±27.12<br>-1.80±16.77 | 119.48±25.05<br>.2.7±17.49 |
| Propanoid acid borneol ester (n=10) | 18mg/kg | 128.25±23.86 | 128.34±28.96<br>-0.06±12.42 | 120.90±26.54<br>-5.49±14.53 | 117.76±22.90<br>-7.74±12.22’ | 106.97±32.22<br>-1634±19.90’ | 104.23±32.16<br>-18.68±1821’’ | 113.34±24.47<br>-10.88±16.04’ |
| Propanoid acid borneol ester (n=10) | 9mg/kg | 132.26±20.00 | 131.10±24.38<br>-1.14±6.68 | 127.85±23.92<br>3.57±7.14’ | 122.58±23.46<br>-7.38±8.66 | 115.46±23.90<br>-12.63±11.39" | 120.90±31.83<br>-8.80±16.65 | 120.85±32.13<br>-9.19±14.65’ |
| Propanoid acid borneol ester (n=10) | 4.5mg/kg | 129.06±22.13 | 126.54±25.94<br>-1.96±11.16 | 128.80±24.39<br>-0.05±10.13 | 129.90±23.86<br>0.93±11.47 | 135.97±22.80<br>5.64±8.91 | 131.95±25.10<br>2.56±13.11 | 128.62±28.30<br>-0.49±12.28 |
| Verapamil control (n=10) | 11.4mg/kg | 137.10±28.79 | 115.30±19.35<br>-14.92±9.40" | 98.48±16.13<br>-26.88±11.21" | 94.47±17.17<br>-29.86±12.10" | 98.49±22.31<br>-26.55±17.97" | 99.15±23.91<br>26.28±17.28’’ | 102.58±27.01<br>-23.82±18.16" |

In comparison with the Tween control group: *$p < 0.05$, **$p < 0.01$

Table 6. Effects on diastolic pressure (mmHg)

| Group | Dose | Before administration | After administration | | | | | |
|---|---|---|---|---|---|---|---|---|
| | | | 5' | 15' | 30' | 60' | 90' | 120' |
| Blank control (n=10) | 2.5mL/kg | 63.92±12.98 | 66.51±13.71<br>4.05±6.32 | 68.12±15.60<br>6.28±10.50 | 6635±18.40<br>2.60±16.02 | 66.54±17.95<br>3.59±15.55 | 66.18±8.51<br>3.24±17.92 | 68.44±20.16<br>6.54±23.33 |
| Tween control (n=10) | 2.5mL/kg | 67.56±14.29 | 66.26±14.25<br>-1.57±8.21 | 67.66±13.23<br>1.46±14.24 | 66.42±13.11<br>0.13±18.94 | 65.95±16.45<br>-1.21±21.94 | 66.05±15.27<br>-0.75±21.63 | 65.27±14.84<br>-1.67±22.83 |
| Propanoid acid borneol ester (n=10) | 18mg/kg | 73.04±11.87 | 72.55±16.58<br>-1.00±14.79 | 68.87 ±14.57<br>-5.45±15.82 | 66.68±11.95<br>-7.68±16.90 | 60.98±16.13<br>-15.66±21.14 | 60.29±15.88<br>-16.74±18.37 | 61.54±13.73<br>-14.64±19.58' |
| Propanoid acid borneol ester (n=10) | 9mg/kg | 69.21±15.19 | 68.02±18.77<br>-2.47±10.80 | 66.18±19.00<br>-5.33±9.64' | 62.90±17.46<br>9.50±10.75' | 59.10±16.07<br>-1430±13.75" | 63.12±19.81<br>-8.83±17.11 | 62.58±22.20<br>-1038±17.60' |
| Propanoid acid borneol ester (n=10) | 4.5mg/kg | 68.47±11.97 | 68.68±15.82<br>-0.08±12.81 | 70.87±15.08<br>3.38±12.17 | 71.78±15.67<br>4.78±14.39 | 76.78±14.34<br>12.13±9.39 | 74.71±18.13<br>8.47±17.66 | 72.46±20.46<br>4.39±16.80 |
| Verapamil control (n=10) | 11.4mg/kg | 75.39±19.27 | 59.69±12.55<br>-19.04±12.70'' | 50.08±9.88<br>-31.18±15.04'' | 48.00±9.98<br>-33.98±15.67" | 48.81±11.47<br>-3254±19.02'' | 49.38±13.27<br>-32.10±18.74'' | 51.84±13.93<br>-29.18±17.73" |

In comparison with the Tween control group: *p<0.05, **p < 0.01

Table 7. Effects on mean arterial pressure (mmHg)

| Group | Dose | Before administration | After administration | | | | | |
|---|---|---|---|---|---|---|---|---|
| | | | 5’ | 15’ | 30’ | 60’ | 90’ | 120’ |
| Blank control (n=10) | 2.5mL/kg | 81.80±15.30 | 84.93±16.35<br>3.81±6.04 | 86.66±18.78<br>5.64±9.78 | 83.87±22.97<br>1.26±15.60 | 83.96±22.23<br>1.82±12.68 | 83.59±23.20<br>1.51±15.15 | 87.13±25.01<br>6.04±22.99 |
| Tween control (n=10) | 2.5mL/kg | 86.51±18.19 | 85.05±18.38<br>-1.45±7.32 | 86.69±16.88<br>1.28±12.05 | 84.92±16.37<br>-0.32±16.30 | 84.24±20.76<br>-1.882±19.29 | 84.38±19.01<br>-1.26±19.14 | 83.34±18.04<br>-2.22 ±20.12 |
| Propanoid acid borneol ester (n=10) | 18mg/kg | 91.44±15.44 | 91.15±20.31<br>-0.58±13.53 | 86.21±18.29<br>-5.52±14.99’ | 83.71±15.31<br>-7.79±14.45 | 76.31±21.36<br>-16.01±20.44 | 74.94±21.07<br>-17.64±18.17 | 78.81±17.04<br>-12.96±17.66’ |
| Propanoid acid borneol ester (n=10) | 9mg/kg | 90.23±16.25 | 89.05±20.26<br>-1.79±8.69 | 86.74±20.25<br>-4.42±8.30’ | 82.79±18.98<br>-8.42±9.56 | 77.89±18.11<br>13.46±12.43’ | 82.38±23.18<br>-8.77±16.66 | 82.00±25.04<br>-9.71±15.95’ |
| Propanoid acid borneol ester (n=10) | 4.5mg/kg | 88.67±14.74 | 87.97±18.89<br>-1.01±11.95 | 90.18±17.91<br>1.66±11.07 | 91.15±17.95<br>2.91±12.81 | 96.51±16.69<br>8.95±8.95 | 93.79±20.11<br>5.62±15.30 | 91.18±22.76<br>2.06±14.37 |
| Verapamil control (n=10) | 11.4mg/kg | 95.96±22.22 | 18.22±14.31<br>-17.07±11.03’’ | 66.21±11.57<br>-29.16±13.09’’ | 63.49±11.93<br>-32.04±13.81’’ | 65.37±14.57<br>-29.68±18.40’’ | 65.97±16.34<br>29.33±17.82’’ | 68.75±17.93<br>-26.65±17.62" |

In comparison with the Tween control group: *p < 0.05, **p < 0.01

Table 8. Effects on left intraventricular pressure (mmHg)

| Group | Dose | Before administration | After administration | | | | | |
|---|---|---|---|---|---|---|---|---|
| | | | 5' | 15' | 30' | 60' | 90' | 120' |
| Blank control (n=10) | 2.5mL/kg | 124.84±4.88 | 129.99±16.77<br>2.48±4.20 | 128.87±18.64<br>3.05±5.46 | 126,10±22.02<br>0.51±8.27 | 126.30±21.69<br>0.75±7.10 | 125.79±22.94<br>0.27±8.10 | 128.12±4.71<br>2.02±10.87 |
| Tween control (n=10) | 25mL/kg | 126.46±14.18 | 125.76±13.38<br>-0.41±4.07 | 127.22±98<br>0.87±6.22 | 125.33±14.26<br>-0.61±8.37 | 11.4.91±16.52<br>-1.07±9.12 | 12432±15.43<br>-1.45±9.18 | 122.50±15.26<br>2.83±9.80 |
| Propanoid acid borneol ester (n-10) | 18mg/kg | 131.33±6.79 | 131.90±18.96<br>-0.01±7.29 | 126.28±18.51<br>-4.15±8.95 | 124.81±18.94<br>-5.46±7.09 | 120.67±23.43<br>-8.71±11.64 | 117.9L±22.68<br>-8.81±11.60* | 119.85±22.26<br>-9.13±13.16** |
| Propanoid acid borneol ester (n=10) | 9mg/kg | 128.29±18.38 | 128.87±25.63<br>-0.03±8.26 | 127.06±25.57<br>-1.37±9.10 | 123.59±25.06<br>-4.07±8.57 | 119.77±23.09<br>-6.84±8.75* | 122.40±26.58.<br>-4.88±11.37 | 121.85±26.36<br>-5.47±9.76 |
| Propanoid acid borneol ester (n=10) | 4.5mg/kg | 128.68±20.18 | 128.67±18.24<br>038±6.72 | 130.13±18.12<br>1.51±6.25 | 129.67±17.48<br>1.22±7.07 | 134.39±19.12<br>4.73±6.55 | 130.91±17.22<br>2.39±9.41 | 129.15±15.33<br>0.79±7.76 |
| Verapamil control (n=10) | 11.4mg/kg | 137.79±18.96<br>-6.24±79** | 128.71±14.20 | 120.22±12.98<br>-12.21±7.42** | 116.59±13.59<br>-14.73±8.91** | 115.68±15.37<br>-15.14±12.14** | 115.10±16.60<br>-15.59±12.52** | 118.39±16.55 **<br>-13.34±11.53 |

In comparison with the Tween control group: *p<0.05, **p <0.01

Table 9. Effects on the change-rate of left intraventricular pressure, MAX (mmHg/s)

| Group | Dose | Before administration | After administration | | | | | |
|---|---|---|---|---|---|---|---|---|
| | | | 5' | 15' | 30' | 60' | 90' | 120' |
| Blank control (n=10) | 1,5mL/kg | 5121.1±940.7 | 5300.2±1071.0<br>3.5±8.2 | 5363.7±1193.1<br>4.6±10.9 | 5258.2±1366.8<br>1.7±13.5 | 5139.2±926.2<br>0-9±10.4 | 5094.3±946.1<br>0.2±12.7 | 5408.2±1342.3<br>5.1±14.8 |
| Tween control (n=10) | 2.5mL/kg | 5246.5±14.18 | 5252.7±13.38<br>1.3±12.6 | 5408.4±12.98<br>4.5±18.3 | 5318.1±14.26<br>2.5±21.3 | 5269.2±16.52<br>1.6±21.5 | 5295.0±15.43<br>2.0±20.2 | 5205.3±15.26<br>0.2±21.8 |
| Propanoid acid borneol ester (n=10) | 18mg/kg | 5695.0±1086.8 | 5694.7±1264.7<br>-0.3±9.3 | 5470.3±1416.7<br>-4.7±11.8* | 5186.9±1177.3<br>-8.9±11.5* | 4905.3±1047.7<br>-12.4±18.7* | 4676.5±941.1<br>-16.7±15.5" | 5012.6±777.5<br>-10.9±12.4** |
| Propanoid acid borneol ester (n=10) | 9mg/kg | 5433.8±1200.0 | 5388.9±1189.0<br>-0.7±6.7 | 5321.6±1133.0<br>-1.6±7.4 | 5179.1*1270.9<br>-5.0±7.8 | 4966-9±1297.4<br>-9.2±10.0' | 5113.5±1356.4<br>-6.4±13.2 | 4959.8±1399.0<br>-9.7±112* |
| Propanoid acid borneol ester (n=10) | 4.5mg/kg | 5380.2±913.7 | 5429.3±1279.7<br>0.4±11.0 | 5435.9±1216.6<br>0.9±11.4 | 5408.8±1352.2<br>0.1±13.5 | 5760.1±1393.5<br>7.1±18.3 | 5706.1±1390.6<br>7.6±26.1 | 5512.7±1250.1<br>3.6±20.4 |
| Verapamil control (n=10) | 11.4mg/kg | 5455.9±418.1 | 4857.7±1002.4<br>-9.7±7.0** | 42843±793.2<br>-19.6±11.8** | 4152.8±901.5<br>-22.1±14.0** | 3919.4±784.0<br>25.4±17.3** | 3759.0±814.5<br>-28.1±19.0** | 4044.6±844.9<br>23.1±18.4** |

In comparison with the Tween control group: *p < 0.05> **p < 0.01

Table 10. Effects on the change-rate of left intraventricular pressure, -MAX (mmHg/s)

| Group | Dose | Before administration | After administration | | | | | |
|---|---|---|---|---|---|---|---|---|
| | | | 5' | 15' | 30' | 60' | 90' | 120' |
| Blank control (n=10) | 2.5mL/kg | -3790.6±596.7 | -3865.0±679.8<br>1.8±4.8 | -3891.±775.2<br>2.3±8.0 | -3879.0±06.5<br>1.3±11.4 | -3881.4±781.9<br>2.0±10.4 | -3858.7±715.1<br>1.8±10.9 | -3985.4±899.9<br>4.4±13.8 |
| Tween control (n=10) | 2.5mL/kg | -3753.7±L603.1 | -3690.5±526.4<br>-1.4±2.7 | -3767.3±551.9<br>0.9±8.5 | -3692.4±698.4<br>-1.0±16.2 | -3673.8±121.5<br>-1.6±6.5 | -3658.5±653.0<br>-1.8±16.4 | -3573.1±702.5<br>-4.4±5.4 |
| Propanoid acid borneol ester (n=10) | 18mg/kg | -4092.0±905.4 | -4103.4±981.2<br>0.0±7.8 | -3991.9±948.6<br>2.4±93. | -3923.8±879.8<br>-3.9±8.2 | -3744.3±851.5<br>-7.9±14.0* | -3659.3±806.3<br>-9.2+16.9 | -3692.1±863.3<br>-8.8±16.9 |
| Propanoid acid borneol ester (n=10) | 9mg/kg | -3881±735.6 | -3908.5±1013.0<br>0.0±11.2 | -3792.8±979.5<br>-2.9±10.2 | -3696.8±997.3<br>-5.5±10.8 | -3569.5±857.8<br>-8.3±10.2* | -3629.7±013.3<br>-72±13.3 | -3541.7±917.7<br>-9.2±11.3* |
| Propanoid acid borneol ester (n=40) | 4.5mg/kg | -4246.669.3 | -4152.9±764.8<br>2.2±8.2 | -4141.1±755.6<br>-2.4±9.1 | -4090.5±704.6<br>-3.6±6.5 | -4146.8±820.5<br>-2.4±10.2 | -4156.0±947.7<br>-2.2±15.9 | -3995.2±837.9<br>-5.6±14.3 |
| Verapamil control (n=10) | 11.4mg/kg | -4215.6±1058.0 | -3812.9±779.3<br>-8.5±6.6** | -3548.9±707.7<br>14.5±9.9** | -3416.6±767.5<br>-17.8±11.8** | -3436.8±691.1<br>-16.2±16.0** | -3247.4±773.2<br>20.9±16.9** | -3452.2±630.9<br>-16.3±11.4** |

In comparison with the Tween control group: *p <0.05, **p < 0.01

Table 11. Effects on area of myocardial infarction in rats

| Group | Dose (mg/kg) | Animal number | Ventricle weight(g) | Non-infarct regionweight (g) | Infarct region weight (g) | Percentage (%) |
|---|---|---|---|---|---|---|
| Sham operation | / | 10 | 0.66±0.03 | | | |
| Model control | / | 10 | 0.68±0.05 | 0.45±0.03 | 0.23±0.04 | 33.77±4.31 |
| bornyl salvianate ester | 10 | 10 | 0.68±0.09 | 0.47±0.07 | 0.20±0.05 | 30.09±5.69 |
| bornyl salvianate ester | 20 | 10 | 0.68±0.08 | 0.49±0.09 | 0.18±0.05* | 25.73±6.35** |
| bornyl salvianate ester | 40 | 10 | 0.63±0.07 | 0.45±0.06 | 0.18±0.04* | 28.95±5.57* |
| Verapamil | 10 | 10 | 0.65±0.09 | 0.46±0.08 | 0.18±0.04* | 27.60±5.03** |
| In comparison with the model control group: *P<0.05, **P<0.01 | | | | | | |

Table 12. Effects on the activities of serum LDH and CK in rats with myocardial ischemia

| Group | Dose (mg/kg) | Animal number | CK (U/L) | CK-MB (U/L) | LDH (U/L) |
|---|---|---|---|---|---|
| Sham operation | / | 10 | 653.1±164.9** | 565.3±122.2** | 1091.7±259.7** |
| Model control | / | 10 | 1058.5±150.0 | 1422.6±182.3 | 2351.4±220.83 |
| bornyl salvianate ester | 10 | 10 | 1214.2±160.2* | 1267.0±274.4 | 1663.7±374.43** |
| bornyl salvianate ester | 20 | 10 | 894.5±176.3* | 1074.1±116.8** | 2022.7±202.63** |
| bornyl salvianate ester | 40 | 10 | 778.0±177.1** | 906.3±99.9** | 1712.5±209.1** |
| Verapamil | 10 | 10 | 752.2±102.8** | 776.6±127.6** | 1160.8±145.0** |
| In comparison with the model control group: *P<0.05, **P<0.01 | | | | | |

Table 13. Effects on the activities of serum SOD and MDA in rats with myocardial ischemia

| Group | Dose (mg/kg) | number | SOD(U/mL) | MDA(nmol/mL) |
|---|---|---|---|---|
| Sham operation | / | 10 | 155.08±21.45** | 6.86±67* |
| Model control | / | 10 | 95.46±26.05 | 18.20±12.10 |
| bornyl salvianate ester | 10 | 10 | 117.66±31.97 | 21.69±27.94 |
| bornyl salvianate ester | 20 | 10 | 111.32±20.36 | 10.60±11.156 |
| bornyl salvianate ester | 40 | 10 | 119.63±23.53* | 9.43±6.57 |
| Verapamil | 10 | 10 | 126.16±28.10* | 11.86±5.93 |
| In comparison with the model control group: *P<0.05, **P<0.01 | | | | |

Table 14. Effects on the ST-T segment of ECG in rats with myocardial ischemia

| Group | Dose (mg/kg) | Animal Number | Elevation of ST-T segment (mV) | | |
|---|---|---|---|---|---|
| | | | Before modeling | 0min after modeling | 24h after modeling |
| Sham operation | / | 10 | 0.18±0.08<br>Change rate(%) | 0.15±0.25<br>140.08±161.56 | 0.12±0.06<br>22.34±21.51** |
| Model control | / | 10 | 0.20±0.09<br>Change rate(%) | 0.19±0.41<br>155.67±158.10 | 0.23±0.10<br>92.67±91.35 |
| bornyl salvianate ester | 10 | 10 | 0.21±0.06<br>Change rate(%) | 0.24±0.24<br>95.00±78.34 | 0.17±0.09<br>49.00±36.27 |
| bornyl salvianate ester | 20 | 10 | 0.17±0.07<br>Change rate(%) | 0.13±0.16<br>91.61±116.85 | 0.11±0.10<br>60.77±39.69 |
| bornyl salvianate ester | 40 | 10 | 0.21±0.06<br>Change rate(%) | 0.36±0.17<br>103.87±148.26 | 0.18±0.07<br>29.01±18.99* |
| Verapamil | 10 | 10 | 0.23±0.04<br>Change rate(%) | 0.23±0.12<br>34.36±35.86* | 0.18±0.04<br>19.17±20.94* |
| In comparison with the model control group: *P<0.05, **P<0.01 | | | | | |

## Claims

**1.** A compound of the formula (I):

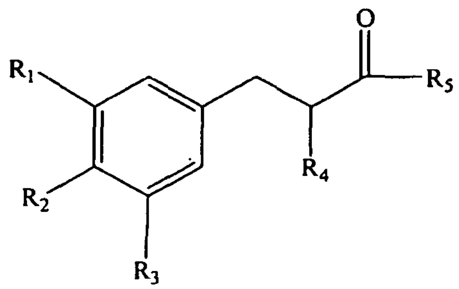

(I)

wherein $R_1$ and $R_2$ are OH; and $R_3$ is H;
$R_4$ is OH;
$R_5$ is

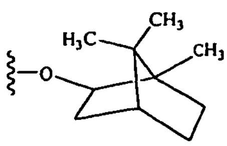

.

**2.** A process for synthesizing a compound according to claim 1, which comprises reacting a compound of formula (III) with a compound of formula (IV) or a hydrate thereof in the presence of a catalyst:

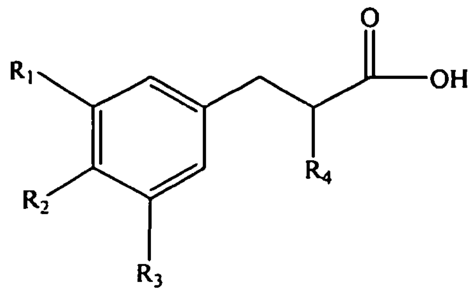

$R_5$-H

(III) (IV)

wherein $R_1$, $R_2$, $R_3$, $R_4$ and $R_5$ have the same meanings as in the formula (I).

**3.** The process according to claim 2, wherein the catalyst is concentrated $H_2SO_4$, silicotungstic acid, phosphomolybdic acid, p-toluene sulfonic acid, $S_2O_8^{2-}$ / $ZrO_2$, aluminum trichloride, zinc chloride and/or magnesium chloride.

**4.** The process according to claim 2, wherein the reaction is performed in a solvent.

**5.** The process according to claim 4, wherein the solvent is ethyl acetate, dichloromethane, tetrahydrofuran, acetone, toluene, 1,4-dioxane or N,N-dimethylformamide, alone or in any combination.

**6.** A compound according to claim 1 for use in the prevention or treatment of cardiovascular or cerebrovascular diseases.

**Patentansprüche**

**1.** Verbindung der Formel (I):

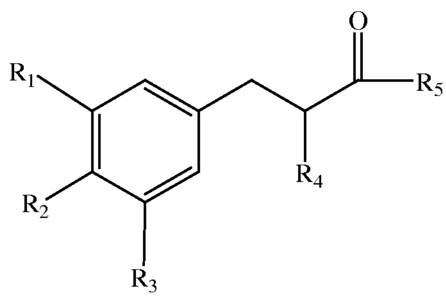

(I)

wobei $R_1$ und $R_2$ OH sind; und $R_3$ H ist;
$R_4$ OH ist;
$R_5$

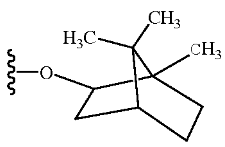

ist.

**2.** Verfahren zur Herstellung einer Verbindung gemäß Anspruch 1, umfassend die Reaktion einer Verbindung der

Formel (III) mit einer Verbindung der Formel (IV) oder einem Hydrat davon in Gegenwart eines Katalysators:

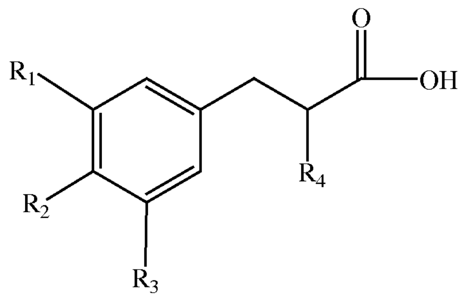

(III)

$R_5$-H

(IV)

wobei $R_1$, $R_2$, $R_3$, $R_4$ und $R_5$ dieselben Bedeutungen wie in Formel (I) haben.

**3.** Verfahren gemäß Anspruch 2, wobei der Katalysator konzentrierte $H_2SO_4$, Kieselwolframsäure, Phosphormolybdänsäure, p-Toluolsulfonsäure, $S_2O_8^{2-}/OZrO_2$, Aluminiumtrichlorid, Zinkchlorid und/oder Magnesiumchlorid ist.

**4.** Verfahren gemäß Anspruch 2, wobei die Reaktion in einem Lösungsmittel durchgeführt wird.

**5.** Verfahren gemäß Anspruch 4, wobei das Lösungsmittel Ethylacetat, Dichlormethan, Tetrahydrofuran, Aceton, Toluol, 1,4-Dioxan oder N,N-Dimethylformamid, allein oder in irgendeiner Kombination davon ist.

**6.** Verbindung gemäß Anspruch 1 zur Verwendung in der Vorbeugung oder Behandlung von kardiovaskulären oder zerebrovaskulären Erkrankungen.

**Revendications**

**1.** Composé de formule (I) :

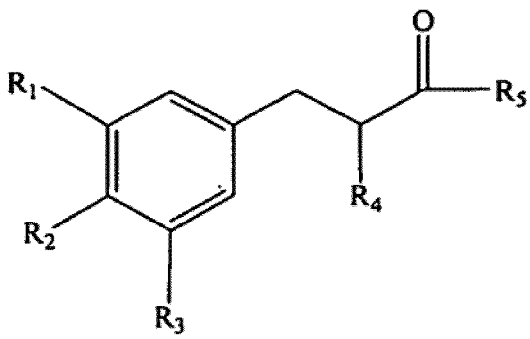

(I)

dans laquelle R1 et R2 sont OH ; et R3 est H ;
$R_4$ est OH ;
$R_5$ test

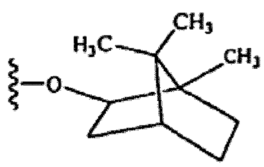

.

**2.** Procédé destiné à synthétiser un composé selon la revendication 1, qui comprend la réaction d'un composé de formule (III) avec un composé de formule (IV) ou un hydrate de celui-ci en présence d'un catalyseur :

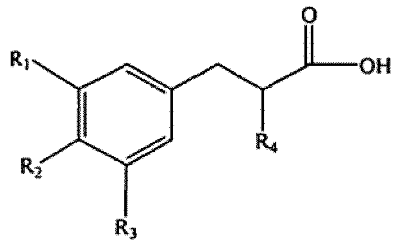

(III)

$R_5$-H

(IV)

dans lesquelles $R_1$, $R_2$, $R_3$, $R_4$ et $R_5$ ont les mêmes significations que dans la formule (I).

**3.** Procédé selon la revendication 2, dans lequel le catalyseur est de l'$H_2SO_4$ concentré, de l'acide silicotungstique, de l'acide phosphomolybdique, de l'acide p-toluènesulfonique, $S_2O_8^{2-}/ZrO_2$, du trichlorure d'aluminium, du chlorure de zinc et/ou du chlorure de magnésium.

**4.** Procédé selon la revendication 2, dans lequel la réaction est effectuée dans un solvant.

**5.** Procédé selon la revendication 4, dans lequel le solvant est l'acétate d'éthyle, le dichlorométhane, le tétrahydrofurane, l'acétone, le toluène, le 1,4-dioxane ou le N,N-diméthylformamide, seul ou en une quelconque combinaison.

**6.** Composé selon la revendication 1 destiné à être utilisé dans la prévention ou le traitement des maladies cardiovasculaires ou vasculaires cérébrales.

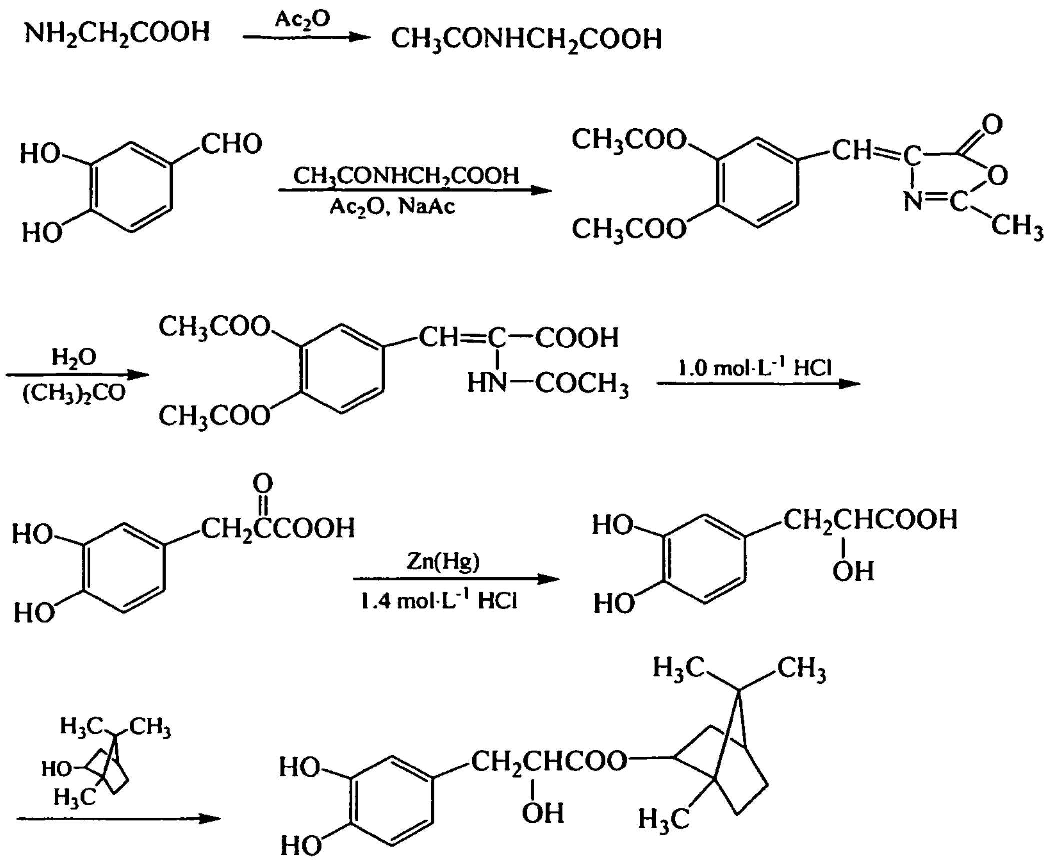
$Ac_2O$
$CH_3CONHCH_2COOH$
$Ac_2O$, NaAc
$H_2O$
$(CH_3)_2CO$
1.0 mol·$L^{-1}$ HCl
Zn(Hg)
1.4 mol·$L^{-1}$ HCl

Fig.1

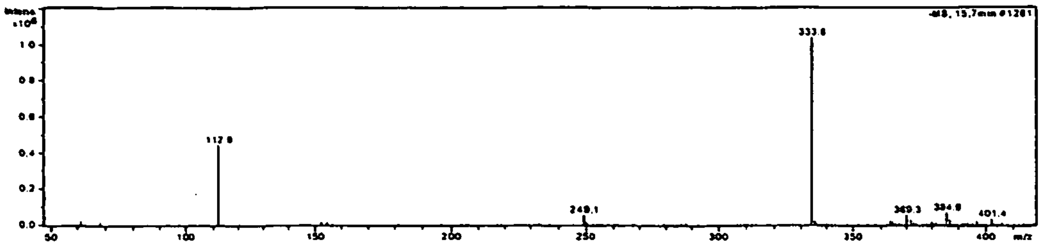
249.1
401.4
0.0
0.2
0.4
50
100
150
200
250
300
350
400
m/z

Fig.2

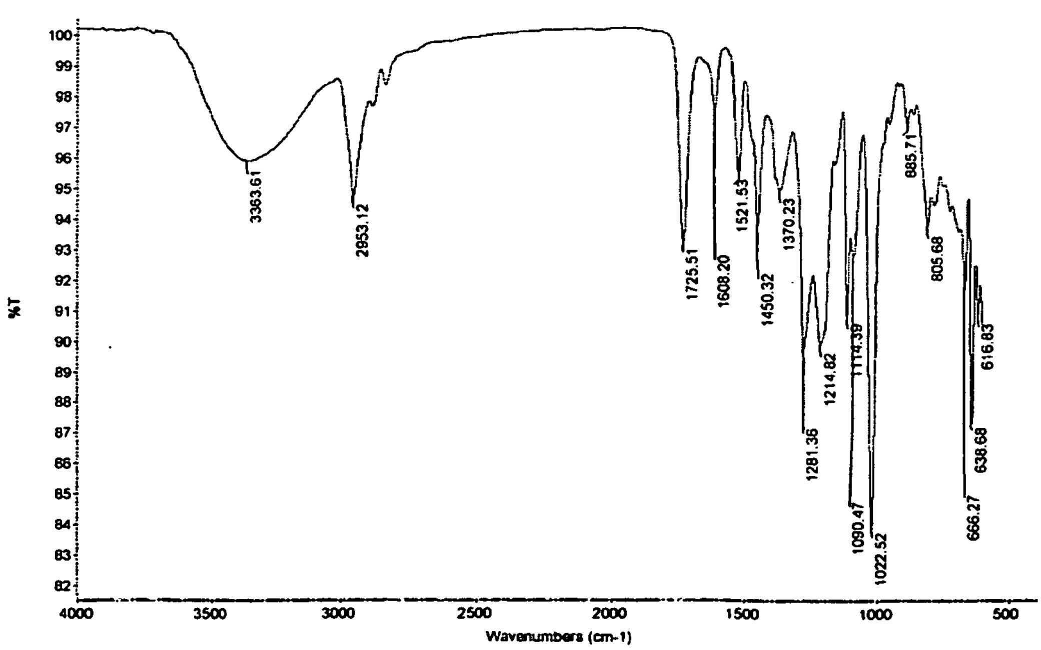
%T
3363.61
2953.12
1725.51
1608.20
1521.53
1450.32
1370.23
1281.36
1214.82
1114.39
1090.47
1022.52
885.71
805.68
666.27
638.68
616.83
4000
3500
3000
2500
2000
1500
1000
500
Wavenumbers (cm-1)

Fig.3

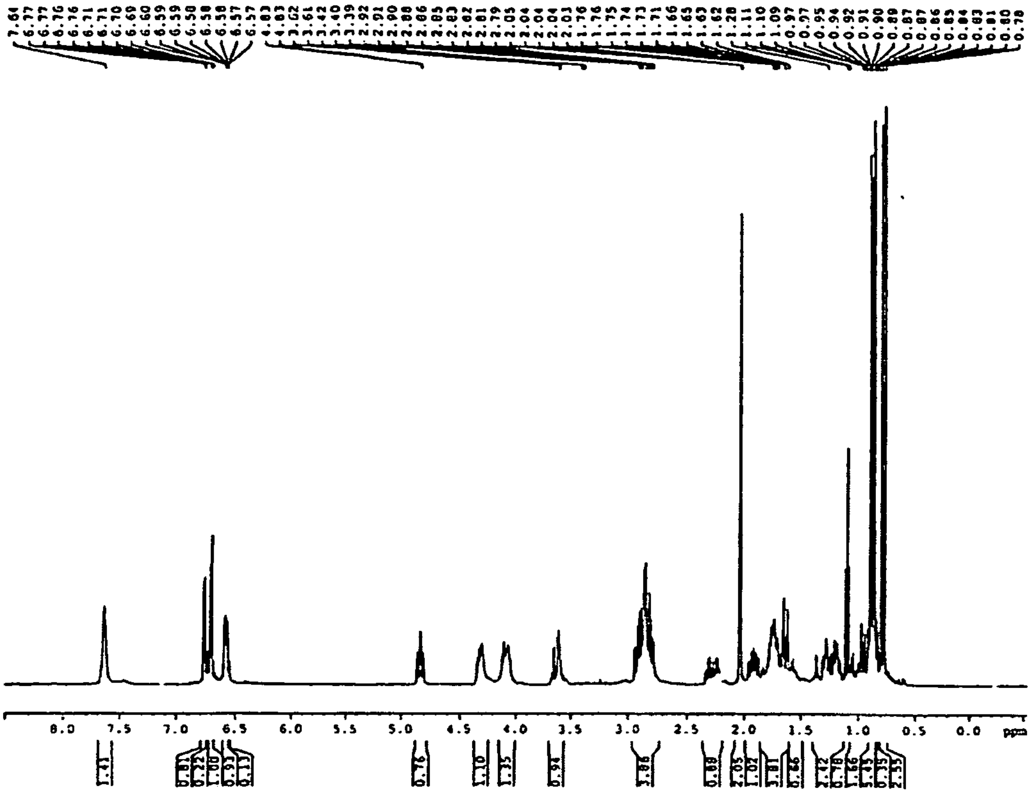

Fig.4

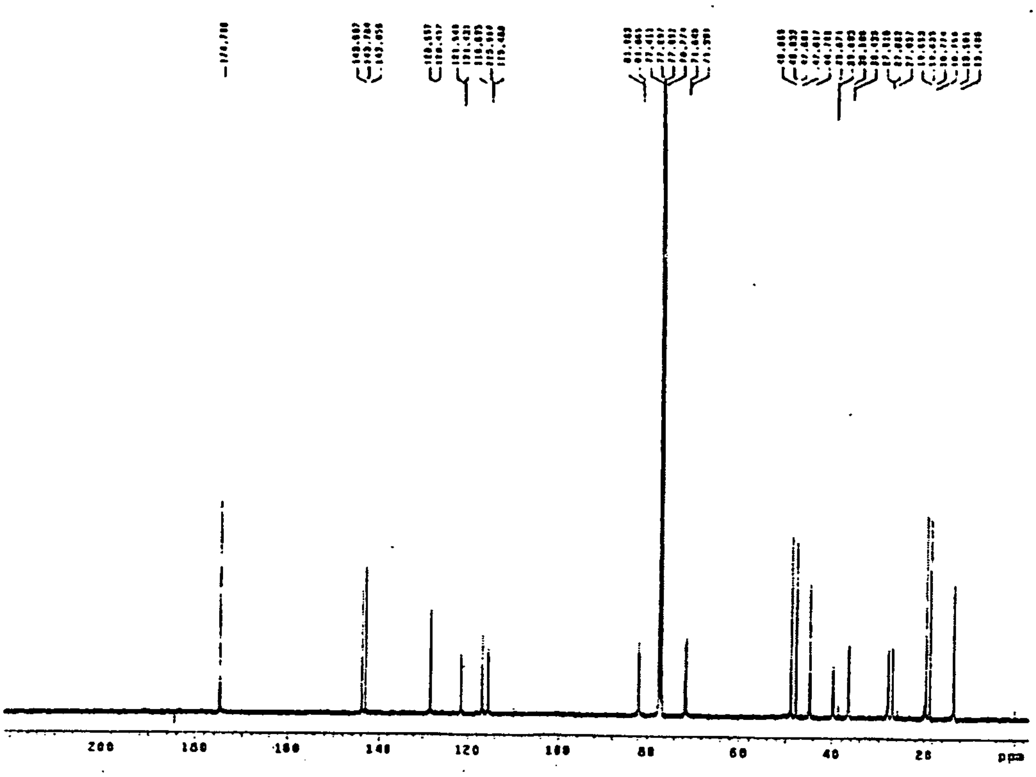

Fig.5